Europäisches Patentamt

European Patent Office

Office européen des brevets

(11) Publication number: **0 510 270 A1**

## EUROPEAN PATENT APPLICATION

(21) Application number: **91303745.3**

(22) Date of filing: **25.04.91**

(51) Int. Cl.⁵: **A61L 25/00**, A61K 37/36, A61F 9/00

(43) Date of publication of application:
**28.10.92 Bulletin 92/44**

(84) Designated Contracting States:
**AT BE CH DE DK ES FR GB GR IT LI LU NL SE**

(71) Applicant: **LINDSTROM, Richard L.**
**1741 Archer Court**
**Plymouth, Minnesota 55447(US)**
Applicant: **SKELNIK, Debra L.**
**PO Box 758, Route 1**
**Cambridge, Minnesota 55008(US)**

(72) Inventor: **LINDSTROM, Richard L.**
**1741 Archer Court**
**Plymouth, Minnesota 55447(US)**
Inventor: **SKELNIK, Debra L.**
**PO Box 758, Route 1**
**Cambridge, Minnesota 55008(US)**

(74) Representative: **Parr, Ronald Edward R.E. Parr**
**& Co.**
**Colman House Station Road**
**Knowle Solihull West Midlands B93 0HL(GB)**

(54) **Viscoelastic solution.**

(57) Viscoelastic solution including a buffered solution, .01-8% chondroitin sulfate, .1-8% hydroxypropyl methyl-cellulose, pH adjusted to 6.0-8.0 at a osmolality between 200-400 mOsmol/L. The buffered solution can be HEPES buffered minimum essential media (MEM), phosphate buffer saline (PBS), buffered balanced salt solution, or TC199. A cell growth factor or cell growth supplement is included in the solution.

EP 0 510 270 A1

## BACKGROUND OF THE INVENTION

1. Field of the Invention - This invention relates to a method of protecting cells or cell coatings during surgery and to compositions suitable for use in that method. In particular, the invention pertains particularly, but not exclusively, to viscoelastic solutions for use during eye surgery to protect cells from mechanical trauma, to maintain or create tissue spaces, to ensure separation and lubrication of tissue surfaces, and/or to permit the manipulation of tissues without mechanical damage by providing cell growth factors, cell supplements and/or basement membrane components that support ocular wound healing. In a preferred form the invention provides a viscoelastic solution which has good coating properties and includes a naturally occurring biocompatible polymer.

2. Description of the Prior Art - There have been numerous prior art solutions such as Healon, a non-inflammatory, high molecular weight fraction of sodium hyaluronate.

A viscoelastic solution is described in U.S. Patent No. 4 713 375 to Lindstrom and Skelnik, issued on December 15, 1987.

## SUMMARY OF THE INVENTION

In a first aspect the present invention provides a viscoelastic composition for use in protecting cells or cell coatings during eye surgery, which comprises:

a. a buffered solution which is at least one of: a balanced salt solution, a HEPES-buffered minimum essential medium, a phosphate-buffered saline and TC 199;

b. at least one of: hydroxypropyl methylcellulose, carboxypropyl methylcellulose, a cellulose gum, dextran and dextran sulfate;

c. chondroitin sulfate; and

d. at least one growth factor or growth supplement; the composition having a pH of 6.0 - 8.0 and an osmolality of 200 - 400 mOsm/L.

In a second aspect the present invention provides a method of protecting cells or cell coatings during eye surgery, which comprises bringing the cells or cell coatings into contact with a viscoelastic composition according to the first aspect of the invention.

For the sake of convenience, the invention is described below with particular reference to the use of hydroxypropyl methylcellulose as the carbohydrate ingredient (b) of the viscoelastic composition.

In a third aspect the present invention provides a viscoelastic solution which utilises a buffered Ph neutral solution as a base and includes the attributes of the combination of chondroitin sulfate and hydroxypropyl methylcellulose. A cell growth factor, cell growth supplement or basement membrane component is also added to the solution.

One significant aspect and feature of the present invention is a viscoelastic solution which provides better viscosity including the viscous properties of the hydroxypropyl methylcellulose and the lubricating properties of chondroitin sulfate.

Another significant aspect and feature of the present invention is providing a buffered pH neutral solution as a base for the viscoelastic solution.

A further significant aspect and feature of the present invention is a viscoelastic solution which provides cell protection and cell coating during eye surgery. The solution provides maintenance of the tissue space, the chondroitin sulfate lubricates the tissue while the hydroxypropyl methylcellulose provides tissue manipulation.

A still further significant aspect and feature of the present invention is a viscoelastic solution with a cell growth factor, cell growth supplement, or basement membrane component that supports corneal wound healing.

## DESCRIPTION OF PREFERRED EMBODIMENTS

Viscoelastic solution includes a buffered solution, .1-8% hydroxypropyl methylcellulose and .01-8% chondroitin sulfate, pH adjusted to 6.0-8.0, and having an osmolality of 200-400 mOsm/L. The buffered solution can be selected from HEPES buffered minimum essential media (MEM), phosphate buffer saline (PBS), buffered salt solution, or tissue culture medium 199. The hydroxypropyl methylcellulose can be substituted with either carboxypropyl methylcellulose or a cellulose gum, dextran or dextran sulfate. Preferably, the hydroxypropyl methylcellulose is present at a concentration of .01-10% while the chondroitin sulfate can be present at a concentration of .01-10 percent by volume. The solution can be introduced into the eye during surgery to protect cells from trauma, to provide lubrication during the procedure, and to promote ocular wound healing.

Cell growth factors or growth supplements which can be used are:

1. Fibroblastic growth factor (FGF), a single chain polypeptide, isolated and purified from the pituitary, human (hFGF) fibronectin or bovine fibronectin (bFGF), in either the acidic or basic forms. The molecular weight range is 14,000 to 16,000. This factor has been demonstrated mitogenic in vitro to a wide variety of cells comprising mesoderm and neuroectoderm tissue.

This also includes synthetic formulated FGF basic peptides consisting of: (1-24)

**Pro-Ala-Leu-Pro-Glu-Asp-Gly-Gly-Ser-Gly-**

**Ala-Phe-Pro-Pro-Gly-His-Phe-Lys-Asp-Pro-**

**Lys-Arg-Leu-Try**

and synthetic formulated FGF acidic peptides consisting of: (1-11)

**Phen-Asn-Leu-Pro-Leu-Gly-Asn-Tyr-Lys-Lys-**

**Pro**

The fibroblastic growth factor can be used at concentrations of .1ng/ml - 100mg/ml.

2. Endothelial Cell Growth Factor (ECGF), prepared from the hypothalamus as a lyophilized extract. This growth supplement has been demonstrated mitogenic in vitro to a wide variety of endothelial cells; i.e., human corneal endothelial cells, human umbilical vein endothelial cells, and mouse Balb/c fibroblasts.

The Endothelial Cell Growth Factor can be used at concentrations of 200ug/ml - 500mg/ml.

3. Urogastrone or Epidermal Growth Factor (EGF), a single chained polypeptide, is composed of 53 amino acids, containing 3 disulfide bonds and has been isolated from mouse submaxillary glands (mEGF) and human urine (hEGF). This growth factor has been demonstrated to be mitogenic in vitro for a wide variety of cells of ectodermal and mesodermal origin.

This also includes synthetic mouse EGF:

**Asn-Ser-Tyr-Pro-Gly-Cys-Pro-Ser-**

**Ser-Tyr-Asp-Gly-Tyr-Cys-Leu-Asn-**

**Gly-Gly-Val-Cys-Met-His-Ile-Glu-**

**Ser-Leu-Asp-Ser-Tyr-Thr-Cys-**

**Asn-Cys-Val-Ile-Gly-Tyr-Ser-Gly-**

**Asp-Arg-Cys-Gln-Thr-Arg-Asp-**

**Leu-Arg-Trp-Trp-Glu-Leu-Arg**

And synthetic EGF [Cys(Acm) 20'31] (20-31)

**Cys-(Acm)-Met-His-Ile-Glu-Ser-Leu-Asp-Ser-**

**Tyr-Thr-Cys(Acm)**

The Epidermal Growth Factor can be used at concentrations of .1ng/ml - 100mg/ml

4. Bovine pituitary extract (BPE), an aqueous extract of bovine or human pituitary glands. This growth supplement has been demonstrated mitogenic in vitro to a wide variety of epithelial cells; i.e., human corneal epithelium, human epidermal keratinocytes

The Bovine Pituitary Extract can be used at concentrations of : .1ng/ml - 500mg/ml

5. Insulin, a polypeptide hormone that functions in the regulation of cellular carbohydrate metabolism and the synthesis of cellular protein, RNA and neutral lipids.

Insulin can be used at concentrations of .1ug/ml - 10mg/ml

6. Transferrin used at .1ug/ml - 10mg/ml

7. Sodium Selenite used at .1ng/ml - 100ug/ml

8. Platelet-derived growth factor (PDGF) a polypeptide stored in platelets and released into serum during clotting, has been shown to be a mitogen for cultured fibroblast cells.

Platelet-derived growth factor can be used at .1ng - 500mg/ml

9. An aqueous extract of bovine or human retinas. This growth supplement has demonstrated mitogenic in vitro to a wide variety of endothelial cells, i.e., human corneal endothelium and human vascular endothelium.

Retinal derived growth factor can be used at .1ng/ml - 10mg/ml

10. Insulin-like growth factor: IGF-1; a single-chain polypeptide with a molecular weight of 7,650 daltons (76 amino acids) and a pI of 8.2 to 8.4. IGF-1, also known as Somatomedin C, is the anabolic basis polypeptide that functions as the mitotic messenger for pituitary growth hormone.

Insulin-like growth factor can be used at .1ng/ml-10mg/ml

11. Transforming Growth Factor - Beta TGFB: TGFB has a molecular weight of 25,000 daltons and is a homodimer composed of two identical 112-amino acid chains.

TGFB can be used at .1ng/ml-10mg/ml

12. Transforming Growth Factor - Alpha:

TGF-alpha can be used at .1ng/ml-10mg/ml

13. Glycosaminoglycans: All used at a concentration of .01-10%:

    1. dermatin sulfate

    2. heparin sulfate

    3. heparan sulfate

    4. keratin sulfate

    5. hyaluronic acid

14. Antioxidants:

    1. ascorbic acid, concentration of .001-10mM

    2. glutathione, concentration of .001-10mM

    3. DL- -tocopherol, concentration of .001-10mM

    4. 2-mercaptoethanol .001-10mM

15. Glycoproteins that promote cellular adhesion and migration (wound healing):

    1. Laminin, a large glycoprotein having a molecular weight of approximately 1,000,000 daltons. The laminin molecule has the shape of an asymmetric cross, comprised of 3B chains of 200,000 daltons each, and one A chain of 400,000 daltons. The chains are held together by disulfide bonds. The single A chain contains a binding site for heparin sulfate. The B chains contain type IV collagen binding sites. The intersection of the three B chains is the locus for cell binding. Laminin provides cells with physiological compatible extracellular matrix that will foster attachment, cytoplasmic spreading and proliferation.

    Laminin can be used at .01ug/ml - 10mg/ml.

    2. Fibronectin is an extracellular matrix-associated glycoprotein composed of two disulfide bonded subunits of 220,000 daltons each. Fibronectin has the potential to interact with several cell surface associated macromolocules including collagen, glycosaminoglycans and cell surface receptors. Fibronectin promotes cell adhesion and migration of human corneal endothelial cells, epithelial cells and fibroblasts.

    Fibronectin can be used at 1ng/ml-10mg/ml

16. Extracellular Matrix Components:

    A. A collagen used in the range 1ng/ml - 1g/ml selected from the group:

        1. Type I collagen;

        2. Type II collagen;

        3. Type III collagen;

        4. Type IV collagen;

        5. Type V collagen; or,

    B. Enactin, used at 1ng/ml - 10mg/ml

    C. Insulin-like growth factors : IGF1 used at .1ng/ml - 10mg/ml

D. Transforming growth factors : TGF alpha and TGF Beta used at .1ng/ml - 10mg/ml

Abbreviations used in the specification have the following meanings:

HEPES    N-2-hydroxyethylpiperazine-N'-ethane sulphonic acid

TC 199    trade mark of a tissue culture medium manufactured by Gibco.

The viscoelastic compositions of this invention can be used in surgery other than eye surgery.

## Claims

1. A viscoelastic composition for use in protecting cells or cell coatings during eye surgery, which comprises:

   a. a buffered solution which is at least one of:
   a balanced salt solution, a HEPES-buffered minimum essential medium, a phosphate-buffered saline and TC 199;

   b. at least one of: hydroxypropyl methylcellulose, carboxypropyl methylcellulose, a cellulose gum, dextran and dextran sulfate;

   c. chondroitin sulfate; and

   d. at least one growth factor or growth supplement;

   the composition having a pH of 6.0-8.0 and an osmolality of 200 - 400 mOsm/L.

2. A composition according to Claim 1, wherein said growth factor comprises a fibroblastic growth factor, for example in acidic or basic form.

3. A composition according to Claim 1, wherein said growth factor comprises an endothelial cell growth factor.

4. A composition according to Claim 1, wherein said growth factor comprises an Urogastrone or Epidermal growth factor.

5. A composition according to Claim 1, wherein said growth factor comprises a bovine pituitary extract.

6. A composition according to Claim 1, wherein said growth factor comprises insulin.

7. A composition according to Claim 1, wherein said growth factor comprises transferrin.

8. A composition according to Claim 1, wherein said growth factor comprises sodium selenite.

9. A composition according to Claim 1, wherein said growth factor comprises platelet-derived growth factor.

10. A composition according to Claim 1, wherein said growth factor comprises a retina extract.

11. A composition according to Claim 1, wherein said growth factor comprises insulin-like growth factor.

12. A composition according to Claim 1, wherein said growth factor comprises transforming growth factor-beta.

13. A composition according to Claim 1, wherein said growth factor comprises transforming growth factor-alpha.

14. A composition according to Claim 1, wherein said growth factor comprises epidermal growth factor (EGF) and insulin.

15. A composition according to Claim 1, wherein said growth factor comprises:

    a. glycosaminoglycan;

    b. an antioxidant;

    c. a glycoprotein; and,

    d. an extracellular matrix component.

**16.** A composition according to Claim 15, wherein said growth factor comprises:

    a. a glycoaminoglycan from the group consisting of:

        i. dermatin sulfate;

        ii. heparin sulfate;

        iii. heparan sulfate;

        iv. keratin sulfate; and

        v. hyaluronic acid;

    b. an antioxidant from the group consisting of:

        i. ascorbic acid;

        ii. glutathione;

        iii. DL-$\alpha$-tocopherol; and

        iv. 2-mercaptoethanol;

    c. a glycoprotein from the group consisting of:

        i. laminin; and

        ii. fibronectin; and

    d. an extracellular matrix component from the group consisting of:

        i. a collagen of:

            type I

            type II

            type III

            type IV

            type V; and

        ii. enactin.

**17.** A viscoelastic solution comprising:

    a. a buffered balanced salt solution;

    b. hydroxypropyl methylcellulose;

    c. chondroitin sulfate; and

    e. Epidermal Growth Factor (EGF);

    the solution having a pH of 6.0 - 8.0 and an osmolality of 200- 400 mOsmol/L.

**18.** A viscoelastic solution comprising:

    a. a buffered balanced salt solution;

    b. 2.75%hydroxypropyl methylcellulose;

    c. 0.5% chondroitin sulfate; and

    d. Epidermal Growth Factor (EGF) (range 0.1ng/ml- 100mg/ml);

    the composition having a pH of 6.0 - 8.0 and an osmolality of 200 - 400 mOsmol/L.

**19.** A viscoelastic solution according to Claim 17, which includes insulin.

**20.** A viscoelastic solution according to Claim 18, which includes insulin in an amount in the range 0.1ug/ml-10mg/ml.

**21.** A surgical solution comprising:

    a. a viscoelastic solution; and

    b. a growth factor in said viscoelastic solution.

**22.** A method of protecting cells or cell coatings during eye surgery, which comprises bringing the cells or cell coatings into contact with a viscoelastic composition as claimed in any of the preceding claims.

European Patent Office

PARTIAL EUROPEAN SEARCH REPORT
which under Rule 45 of the European Patent Convention shall be considered, for the purposes of subsequent proceedings, as the European search report

Application Number

EP  91 30 3745

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| Y | EP-A-0 240 031  (ALLERGAN)<br>* Page 15, lines 5-7; claims 1-3,12-14 *<br>--- | 1,2,4,9,11-13,17,18 | A 61 L  25/00<br>A 61 K  37/36<br>A 61 F   9/00 |
| D,Y | EP-A-0 213 734  (R.L. LINDSTROM)<br>* Column 2, lines 10-13; claims 1-5 *<br>--- | 1,17,18 | |
| X | EP-A-0 378 852  (MERCK)<br>* Page 2, lines 7,8; claim 1 * | 21 | |
| Y | | 2 | |
| Y | EP-A-0 312 208  (ETHICON)<br>* Page 4, lines 38-41; page 5, lines 20-29,42-50 *<br>----- | 2,4,9,11,13 | |

TECHNICAL FIELDS
SEARCHED (Int. Cl.5)

A 61 L
A 61 K

## INCOMPLETE SEARCH

The Search Division considers that the present European patent application does not comply with the provisions of the European Patent Convention to such an extent that it is not possible to carry out a meaningful search into the state of the art on the basis of some of the claims.

Claims searched completely: 1-21  Viscoelastic solution for use in eye surgery

Claims searched incompletely:

Claims not searched: 22

Reason for the limitation of the search: Method of eye surgery

Method for treatment of the human or animal body by surgery or therapy (see Art. 52(4) of the European Patent Convention).

| Place of search | Date of completion of the search | Examiner |
|---|---|---|
| THE HAGUE | 10-12-1991 | PELTRE CHR. |